# EUROPEAN PATENT APPLICATION

(11) **EP 3 366 194 A1**
(43) Date of publication of application: **29.08.2018**
(21) Application number: 16856976.2
(22) Date of filing: 19.10.2016
(51) Int. Cl.: A61B 1/24, A61B 13/00, A61B 17/94

(54) **MOUTH PROTECTOR DEVICE WITH TONGUE DEPRESSOR**

(30) Priority: 21.10.2015 ES 201531508
(71) Applicant: Fundacion para la Investigacion Biomedica Del Hospital Gregorio Marañon, 28007 Madrid (ES)
(72) Inventor: RODRÍGUEZ BERNAL, Guillermo Juan, 28007 Madrid (ES); CASANOVA BAREA, Javier, 28007 Madrid (ES)
(74) Representative: Pons
(86) International application number: PCT/ES2016/070741
(87) International publication number: WO 2017/068221

(57) **Abstract**

Mouth protector device with tongue depressor, comprising a first cover (1) for protecting the lip and the teeth of the upper jaw and a second cover (2) for protecting the lip and the teeth of the lower jaw, both covers being connected and articulated in relation to each other by an opening and locking regulator (3), which holds the patient's mouth open for the introduction of exploration or operating instruments through the oral cavity, wherein the second cover (2) has a through groove (4) for the insertion of a semi-rigid tongue depressor (5) which depresses and holds back the tongue. The first (1) and second (2) covers further comprise side extensions (6) for protecting the row of premolars and molars and at least one tape (7) for connecting the rear ends thereof, said tape passing around the rear part of the patient's head, in order to provide the device with increased stability.

## Description

### OBJECT OF THE INVENTION

The present invention is included in the technical field of instruments used for medical examination through the mouth which have a tongue depressor, and of devices to act on patients' respiratory system by gas-based treatment, and it relates in particular to a device to protect the lips and teeth of a patient being examined or operated on through the oral cavity, which incorporates a tongue depressor.

### BACKGROUND OF THE INVENTION

In typical clinical practice there are various operations and diagnostic tests, e.g. endoscopy, fibrobronchoscopy, transoesophageal echography, etc., wherein it is necessary to insert different instruments, both in the digestive tract and in the airways, through the patient's mouth. Hence, numerous devices are known in the state of the art, which are normally limited to protecting the medical material introduced in the mouth and, to a lesser extent, the patient's teeth.

It is also typical that during execution of these diagnostic tests it is necessary to use sedation so that the procedure is better tolerated, which prevents and hinders the patients' normal breathing, largely due to the depressor effect exerted by the drugs on the oropharyngeal and tongue muscles. When typical mouth protectors are inserted in the patient's mouth, they displace the tongue towards the rear part, so that it protrudes and hinders both the patient's correct breathing and the manoeuvre to introduce the exploratory material in question from the oral cavity to the digestive tract or the lower airways.

Furthermore, one of the main reasons for complaint in patients who have been anaesthetized arises from the breakage of teeth due to the use of laryngoscopy for the orotracheal intubation necessary to establish mechanical or assisted breathing for any type of general anaesthesia, as there is no systematic use of protective measures to avoid said breakages. Furthermore, the greater morbimortality of general anaesthesia is associated to complications related to the difficult access to the airways in certain patients.

### DESCRIPTION OF THE INVENTION

The object of the invention consists of a mouth protector device which incorporates a tongue depressor, designed to be inserted in the patient's mouth and hold it open to perform diagnostic tests or surgical operations that require instruments to be inserted through the oral cavity, which avoids the aforementioned problems.

Therefore, the device firstly comprises two structures, preferably made in materials of soft consistency, designed to define an access to the patient's oral cavity by separating the jaws, protecting at the same time the lips and teeth. With said purpose, it covers them by means of cover-type parts with a form similar to the dental arch and a thickness which allows it to go from the outer surface of the lips to the inner surface of the teeth. A first cover protects both the lip and dental arch corresponding to the upper part of the mouth, whilst the second cover is designed to protect the lip and dental arch of the lower part.

Said first and second covers have standardized dimensions of those typical for the mouth of an adult user, but they can be made in any size, even paediatric, and further comprise side extensions for also protecting the rows of premolars and molars both in the mandible or lower jaw and in the upper jaw.

Both covers are articulated in relation to one another by a mechanism, preferably of gear wheel-type, which regulates the degree of separation between them and, therefore, the degree of opening of the oral cavity, so that, once the necessary separation is achieved between jaws to define sufficient access so that the exploration or operating instruments access the inside of the mouth, it locks said opening, fixing the articulation of the structures in the patient's mouth so that they avoid closure, thus decreasing the risk of injuries.

In an alternative embodiment, the mechanism that regulates the degree of separation between covers consists of a saw-tooth type structure made in the rear side ends of the second cover, whereto oscillating tabs made in the corresponding rear side ends of the first cover are fixed.

On the other hand, the second cover, designed to protect the lip and the lower dental arch, has a through-opening in the form of a groove in the centre of its front part, with the dimensions to insert a semi-rigid tongue depressor which, exceeding the upper part of the second cover which protects the lip and the dental arch of the mandible, and following the curvature of the tongue, advances towards the patient's pharynx, and can reach the epiglottic vallecula, depressing in turn the tongue so that this does not obstruct the oral cavity and the oropharynx and, therefore, does not hinder the patient's breathing. Said depressor in this way favours the introduction of diagnostic instruments of different types, such as, for example, endoscopes, fibrobronchoscopes, probes and even a laryngoscope, decreasing the risk of obstructing the airways. The groove also allows anchoring the tongue depressor once the desired depression level is achieved, leaving it fixed and thus facilitating the operations that may be performed.

Each one of the upper and lower protectors may incorporate an adjustable tape or elastic band which connects the rear sides of each protector surrounding the rear part of the head, in this way helping to stabilize the device and preventing undesired mobilizations thereof.

The device thus described, therefore essentially consisting of a teeth and lip protector which incorporates a mouth opening and tongue depressor system, in this way resolves the main problems arising from explorations or operations performed through the mouth, since:
- It avoids the protrusion of the tongue or its displacement against the teeth, thanks to the anchoring of the tongue depressor, before diagnostic or therapeutic tests, and its subsequent fall towards the oropharynx due to the effects of the anaesthetic drugs and the instruments introduced.
- It maximizes the mouth opening when there is a need to introduce devices, which allows access to the oropharyngeal cavity and enables the insertion of instruments such as endoscopes, fibrobronchoscopes, probes of different application and even laryngoscopes, minimizing oral and pharyngeal damage.
- It improves spontaneous breathing through the oral cavity and protects teeth and lips.

All thanks to a device which is simple to place in and remove from the patient's mouth, which also avoids the discomfort typical of having the mouth open during long periods of time. Furthermore, it is devised for a single use, so that it is preferably made in economical, easily-disposable and recyclable materials such as low-density polyethylene or flexible polyvinylchloride, meaning that, in addition to being comfortable, this device is hygienic and safe for the patient.

### DESCRIPTION OF THE DRAWINGS

To complement the description being made and in order to aid towards a better understanding of the characteristics of the invention, in accordance with a preferred example of practical embodiment thereof, a set of drawings is attached as an integral part of said description wherein, with illustrative and non-limiting character, the following has been represented:
Figure 1.- Shows a perspective view of the device placed in the patient's mouth, before inserting a tongue depressor.
Figure 2.- Shows a front view of the device placed in the patient's mouth, with the tongue depressor inserted.
Figure 3.- Shows a side view of a section of the device placed in the patient's mouth, wherein it is observed how it protects both the teeth and the lips, and how the tongue depressor is inserted towards the initial part of the pharynx.
Figure 4.- Shows a side view of the device placed in the patient's mouth, wherein it is observed how it is secured by a tape passing around the rear part of the head.

### PREFERRED EMBODIMENT OF THE INVENTION

A detailed explanation of the example of preferred embodiment of the object of the present invention is provided below, with the aid of the aforementioned figures.

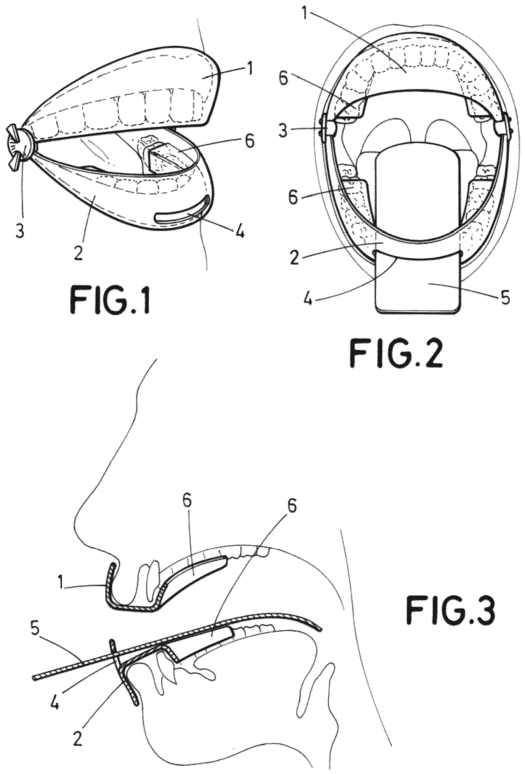

As can be observed in figure 1, the mouth protector device with tongue depressor described below fundamentally comprises a first cover (1) and a second cover (2), connected and articulated in relation to each other by means of an opening and locking regulator mechanism (3), which is inserted in the patient's mouth. Both the first cover (1) and the second cover (2) have the shape, similar to an arch, and the necessary dimensions to cover the patient's lips and dental arches; the first cover (1) covers the upper lip and the dental arch of the upper jaw, whilst the second cover (2) covers the lower lip and the dental arch of the mandible or lower jaw.

The opening and locking regulator mechanism (3), located on the sides of the rear ends of the first cover (1) and the second cover (2), creates an articulated joint between both covers, also regulating in this way the degree of opening of the oral cavity by the separation between the upper and lower jaws. Once said jaws have separated, defining an opening access to the oral cavity with the appropriate dimensions for the introduction of the necessary instruments, the opening and locking regulator mechanism (3) fixes said separation between first cover (1) and second cover (2), thus preventing the closure of the jaws. In a preferred embodiment such as that shown in figure 1, said opening and locking regulator mechanism (3) comprises a gear wheel, wherein each rotation defines a degree of separation between the first cover (1) and the second cover (2) until, once the necessary mouth opening is achieved, it locks the rotation of said wheel, thus fixing the separation between jaws.

As shown in figures 1, 2 and 3, the second cover (2) further comprises a groove (4), preferably made in the centre of its front surface and through towards the inside of the oral cavity, wherethrough a semi-rigid tongue depressor (5) is inserted and with a degree of curvature in its initial end suitable for the lingual and pharyngeal anatomy which, exceeding the upper part of the lower lip and the dental arch of the mandible, as detailed in figure 3, it follows the curvature of the tongue and advances towards the oropharynx, which is the anatomical region which includes the rear third of the tongue and goes from the soft palate to the hyoids, and can even reach the epiglottic vallecula, if required. The groove (4) allows the anchoring of the tongue depressor (5) once it is disposed in the desired position of depression and immobilisation of the tongue, fixing it without the need for fastening by the healthcare professional and thus facilitating exploration operations.

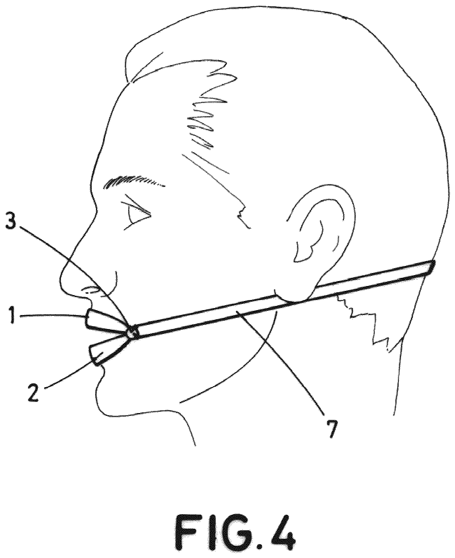

Both the first cover (1) and the second cover (2) further comprise side extensions (6) to cover and protect the row of molar and premolar teeth both of the upper jaw and the lower jaw, and, optionally, an adjustable tape (7) or elastic band made in flexible material which connects the respective rear ends of the first cover (1) and of the second cover (2) surrounding the rear part of the patient's head, as shown in figure 4, in this way stabilizing the device and guaranteeing its immobility.

The first cover (1), the second cover (2) and its side extensions (6), are made in disposable materials of soft consistency which cover without damaging the patient's lips and teeth; in a preferred embodiment, said material is low-density polyethylene which may additionally have a silicone coating in the areas in direct contact with the most fragile parts, such as the lips and the gums.

The mouth protector device with tongue depressor thus described is a hygienic, safe device which is simple to remove from the patient's mouth, which avoids the discomfort of remaining with the mouth open and protects the lips from the damages that usually occur during the explorations and operations performed through the oral cavity. Furthermore, for the case of those patients that must be sedated, it avoids the efforts of the professionals involved to maintain the necessary opening.

## Claims

1. A mouth protector device with tongue depressor for placing in the oral cavity of a patient, comprising:
- a first cover for covering and protecting the upper lip and the dental arch of the upper jaw,
- a second cover for covering and protecting the lower lip and the dental arch of the lower jaw, and
- an opening and locking regulator which articulately joins the first cover and the second cover, and defines an access opening to the inside of the oral cavity,
wherein the mouth protector further comprises:
- a groove made in the second cover through towards the inside of the oral cavity, for the insertion of a tongue depressor, and
- side extensions made in both the first cover and in the second cover, for covering and protecting the rows of molar and premolar teeth both of the upper jaw and the lower jaw.

2. The mouth protector device of claim 1 wherein the opening and locking regulator comprises a gear-wheel type mechanism.

3. The mouth protector device of claim 1 wherein the opening and locking regulator comprises a saw-tooth type structure made in the rear side ends of the second cover whereto are fixed oscillating tabs made in the corresponding rear side ends of the first cover.

4. The mouth protector of claim 1 wherein the first cover, the second cover and its side extensions are made in disposable materials of soft consistency.

5. The mouth protector device of claim 3 wherein the disposable materials of soft consistency comprise low-density polyethylene.

6. The mouth protector device of claim 3 wherein the first cover and the second cover comprise an additional coating in the areas which are in direct contact with lips and gums.

7. The mouth protector device of claim 5, wherein the additional coating comprises silicone.

8. The mouth protector device of claim 1, wherein it additionally incorporates at least one adjustable tape for connecting the respective rear ends of the first cover and of the second cover surrounding the rear part of the patient's head, for immobilizing the device.
